# NEUE EUROPÄISCHE PATENTSCHRIFT

(11) **EP 3 694 352 B2**
(45) Veröffentlichungstag und Bekanntmachung des Hinweises auf die Entscheidung über den Einspruch: **25.11.2026**
(45) Hinweis auf die Patenterteilung: 01.12.2021
(21) Anmeldenummer: 18786274.3
(22) Anmeldetag: 10.10.2018
(51) Int. Cl.: A24F 47/00, A24F 40/44, A24F 40/485, A61M 15/06, A61M 11/04, A61M 15/02

(54) **VERDAMPFEREINHEIT FÜR EINEN INHALATOR, INSBESONDERE FÜR EIN ELEKTRONISCHES ZIGARETTENPRODUKT**
EVAPORATOR UNIT FOR AN INHALER, IN PARTICULAR FOR AN ELECTRONIC CIGARETTE PRODUCT
UNITÉ VAPORISATEUR DESTINÉE À UN INHALATEUR, NOTAMMENT À UN PRODUIT CIGARETTE ÉLECTRONIQUE

(30) Priorität: 13.10.2017 DE 102017123870
(43) Veröffentlichungstag der Anmeldung: 19.08.2020
(73) Patentinhaber: Nicoventures Trading Limited, London WC2R 3LA (GB)
(72) Erfinder: SCHMIDT, Rene, 21244 Buchholz i.d.N. (DE); KESSLER, Marc, 22415 Hamburg (DE); NIEBUHR, Gunnar, 22605 Hamburg (DE); KALAYDZHYAN, Karen, 22607 Hamburg (DE)
(74) Vertreter: D Young & Co LLP
(86) Internationale Anmeldenummer: PCT/EP2018/077601
(87) Internationale Veröffentlichungsnummer: WO 2019/072915

(56) Entgegenhaltungen:
- EP-A1- 3 117 860
- EP-A1- 3 372 096
- WO-A1-2018/083007
- US-A1- 2009 220 222
- US-A1- 2009 224 064
- US-A1- 2015 059 780
- US-A1- 2016 345 630

## Beschreibung

Die vorliegende Erfindung betrifft eine Verdampfereinheit für einen Inhalator, insbesondere für ein elektronisches Zigarettenprodukt, mit einem elektrisch betreibbaren, insbesondere planaren Heizkörper, der eine Einlassseite und eine Auslassseite aufweist, und einer Mehrzahl von Mikrokanälen, die sich jeweils von der Einlassseite zu der Auslassseite durch den Heizkörper erstrecken, wobei der Heizkörper durch Anlegen einer Heizspannung zum Verdampfen von durch die Mikrokanäle geförderter Flüssigkeit eingerichtet ist.

Im Stand der Technik erfolgt die Liquidzufuhr zum Heizkörper typischerweise kapillar mittels eines Dochts. Die verwendeten Dochte haben entlang der Förderrichtung idealerweise eine konstante Förderwirkung. Ist die Förderrate geringer als die geforderte Verdampfungsrate, so trocknet der Docht in direkter Nähe zum Heizkörper aus. Es folgt ein Trockenzug (sogenannter Dry Puff) und Schadstoffe werden freigesetzt.

Im Falle eines planaren Heizkörpers muss der Heizkörper zu jeder Zeit und an jedem Ort durch den Docht möglichst gleichmäßig benetzt werden, um eine konstante Temperaturverteilung und damit gleichmäßige, schadstofffreie Verdampfung über seine Oberfläche zu gewährleisten. EP 3 117 860 A1 offenbart eine Verdampfereinheit für einen Inhalator.

Die Aufgabe der Erfindung besteht darin, eine jederzeit funktionssichere Verdampfereinheit mit hoher thermo-elektromechanischer Stabilität bereitzustellen, mit der die Entstehung von Schadstoffen bei der Verdampfung von Flüssigkeit vermieden werden kann.

Die Erfindung löst diese Aufgabe mit den Merkmalen der unabhängigen Ansprüche.

Erfindungsgemäß ist an der Einlassseite des Heizkörpers eine poröse und/oder kapillare Dochtstruktur angeordnet, die flüssigkeitsleitend mit einem Flüssigkeitsspeicher verbunden oder verbindbar ist. Die Dochtstruktur weist einen sich durch eine Durchgangsöffnung des Trägers erstreckenden Schaft und einen zwischen dem Träger und dem Heizkörper angeordneten umlaufenden Kragen auf. Der Durchmesser des Kragens ist erfindungsgemäß größer ist als der Durchmesser der Durchgangsöffnung des Trägers. Der Kragen kann daher auf dem die Durchgangsöffnung bildenden Teil des Trägers aufliegen und auf diese Weise die Dochtstruktur halten, da der Kragen aufgrund der erfindungsgemäßen Bemaßung nicht durch die Durchgangsöffnung in Richtung Flüssigkeitsspeicher auswandern kann, was die Funktionalität der Verdampfereinheit beeinträchtigen würde.

Vorzugsweise ist mindestens ein Vorspannungs-erzeugendes Klemmelement vorgesehen, das zur Klemmung des Heizkörpers und des Kragens auf den Träger angeordnet und eingerichtet ist. Mittels des Klemmelements wird der der Kragen der Dochtstruktur zwischen dem Heizkörper und dem Träger eingeklemmt und auf diese Weise die Dochtstruktur sicher und unverrückbar in der Verdampfereinheit gehalten. Dabei steht der Kragen über seinen gesamten Umfang über die Durchgangsöffnung des Trägers über, vorzugsweise mit einem Überstand von mindestens 0,1 mm oder mehr. Durch den allseitigen Überstand wird eine gleichmäßige Klemmung erreicht und Leckage vermieden.

In einer besonders bevorzugten Ausführungsform dient das mindestens eine Klemmelement gleichzeitig als Elektrode zur elektrischen Kontaktierung und Versorgung des Heizkörpers. In diesem Fall sind separate Elektroden für die elektrische Kontaktierung des Heizkörpers entbehrlich.

Vorzugsweise sind mindestens zwei Klemmelemente auf gegenüberliegenden Seiten des Heizkörpers vorgesehen, was eine besonders hohe mechanische Stabilität mit relativ geringem Aufwand ermöglicht. In einer bevorzugten Ausführungsform weist das mindestens eine Klemmelement einen den Heizkörper linienförmig kontaktierenden Klemmbügel auf. Aufgrund der Linienkontaktierung zwischen dem Klemmbügel und dem Heizkörper ergibt sich eine ausgezeichnete elektrische Verbindung zwischen dem Klemmelement und dem Heizkörper, bei gleichzeitig idealer thermischer Entkopplung zwischen dem Klemmelement und dem Heizkörper wegen fehlendem Flächenkontakt.

Das Klemmelement kann den Heizkörper seitlich parallel zur Auslassseite und/oder senkrecht auf die Auslassseite und/oder in einer Nut oder Stufe des Trägers klemmen. Die letztgenannte Möglichkeit involviert zwei Kontaktlinien zwischen dem Klemmbügel und dem Heizkörper, was die elektrische Kontaktierung weiter erheblich verbessert. Ein Klemmelement kann auch mehr als einen Klemmbügel aufweisen, insbesondere beliebige zwei oder alle drei Klemmbügel der vorgenannten Art.

In einer vorteilhaften Ausführungsform kann mindestens ein sich durch eine Bohrung des Trägers erstreckender elektrischer Leiter zur elektrischen Kontaktierung des Klemmelements vorgesehen sein, der insbesondere eine Leiterplatte kontaktiert, die auf der von dem Heizkörper abgewandten Seite des Trägers beabstandet angeordnet ist . Es ist aber auch vorteilhaft möglich, dass der Träger selbst als Leiterplatte ausgebildet ist, was die Anzahl der Teile und somit den Herstellungsaufwand reduziert.

Nach alledem wird vorteilhaft ein insbesondere planarer Silizium-Heizkörper bereitgestellt, der die Dochtstruktur so auf den Träger klemmt, dass die Dochtstruktur mechanisch fixiert ist, eine sichere luftundurchlässige hydraulische Kopplung zwischen dem Heizkörper und dem Flüssigkeitsreservoir entsteht, und gleichzeitig die elektrische Ankopplung des Heizkörpers gewährleistet ist. Heizkörper und Reservoir befinden sich an gegenüberliegenden Seiten des Dochts.

Der Kragen kann vorteilhaft ein Teller, ein Flansch oder ein in einer anderen Ebene umlaufender Kragen sein. Im Falle eines Tellers ist die Dochtstruktur somit pilzförmig ausgebildet. Der erfindungsgemäße Kragen ermöglicht eine Klemmung der Dochtstruktur zwischen dem Träger und Heizkörper bei gleichzeitiger Liquidleitung durch die Durchgangsöffnung des Trägers. Der Heizkörper ist durch die Dochtstruktur thermisch vom Träger isoliert, gleichzeitig stellt die Klemmung durch elektrische Leiter auch die Stromversorgung des Heizkörpers dar. Die elektrische Verbindung übt einen Anpressdruck auf Heizkörper und Dochtstruktur aus, welcher thermischen Drücken während der Verdampfung entgegenwirkt.

Wesentlich ist eine flüssigkeitsleitende Anlage zwischen einer Dochtfläche und einer Fläche des Heizkörpers, die den Kragen vorteilhaft einbeziehen kann, aber nicht unbedingt muss. Der Träger kann beispielsweise auch von einer, gegebenenfalls verbreiterten, Gehäusewand des Flüssigkeitsreservoirs ausgebildet sein. In diesem Fall kann ein separater Träger entbehrlich sein.

Mittels der Dochtstruktur kann der Blasenbildung im Einlassbereich des Heizkörpers entgegenwirkt werden. Bläschen, die in den Mikrokanälen des Heizkörpers entstehen, können nicht in den Bereich stromaufwärts von der Einlassseite vordringen und zu einem Trockenlaufen des Einlassbereichs des Heizkörpers, und somit zu einer Funktionsbeeinträchtigung des Verdampfers führen. Etwaige Bläschen im Bereich der Dochtstruktur sind in deren Poren bzw. Kapillaren gefangen und können sich nicht großen Blasen vereinigen. Wichtig ist dabei, dass die Dochtstruktur flächig und kontaktierend an der Einlassseite an dem Heizkörper anliegt und sämtliche Mikrokanäle an der Einlassseite überdeckt, damit einzelne Bläschen, die in den Mikrokanälen entstehen, nicht in der falschen Richtung, nämlich auf der Einlassseite zum Flüssigkeitsspeicher hin, aus den Mikrokanälen austreten können. Vielmehr sorgt die Blockade der Einlassseite durch die erfindungsgemäße Dochtstruktur dafür, dass in den Mikrokanälen entstehende Bläschen in den Mikrokanälen zur Auslassseite hin wandern, wo sie aus den Mikrokanälen ausgetrieben werden und dann keine Probleme mehr bereiten können.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Vorteilhaft ist die Förderrate der Dochtstruktur mindestens so groß ist wie die maximale Verdampfungsrate des Heizkörpers. Damit wird jederzeit eine ausreichende Flüssigkeitsnachführung sichergestellt, so dass ein nachteiliges Leerlaufen des Heizkörpers verhindert wird. Die Verdampfungsrate wird dabei bestimmt durch die Geometrie der Heizkörperstruktur (Volumen vs. Oberfläche) und die Verdampferleistung.

Demnach ist die kapillare Dochtstruktur eingerichtet, um das Liquid gleichmäßig über ihr gesamtes Volumen zum Heizkörper fördern. Förderrate der Dochtstruktur und Verdampfungsrate des Heizkörpers sind so zueinander eingestellt, dass die Förderrate mindestens die Verdampfungsrate bedienen kann. Auf diese Weise wird verhindert, dass während des Verdampfungsvorgangs zu wenig Liquid am Heizkörper anliegt, wodurch dieser austrocknen würde.

Die Dochtstruktur kann aus jedem hinreichend hitzebeständigen, porösen und/oder kapillaren Material mit geeigneter Förderrate bestehen. Vorteilhaft kann die Dochtstruktur ganz oder teilweise bestehen aus Baumwolle, Cellulose, Acetat, Glasfasergewebe, Glasfaserkeramik, Sinterkeramik, keramisches Papier, Alumosilikat-Papier, Metallschaum, Metallschwamm, und/oder einem Verbund von zwei oder mehr der vorgenannter Materialien.

Die Erfindung wird im Folgenden anhand bevorzugter Ausführungsformen unter Bezugnahme auf die beigefügten Figuren erläutert.

Dabei zeigt
- Fig. 1: eine schematische Darstellung eines elektronischen Zigarettenprodukts;
- Fig. 2: eine perspektivische Querschnittsansicht einer Verdampfereinheit;
- Fig. 3: eine schematische Querschnittsansicht einer Verdampfereinheit in einer Ausführungsform der Erfindung;
- Fig. 4, 5: Aufsicht auf den Träger einer Verdampfereinheit von der Seite des Heizkörpers (Figur 4) und von der entgegengesetzten Seite der Flüssigkeitszuführung (Figur 5);
- Fig. 6, 8: Querschnittsansichten einer Verdampfereinheit in weiteren Ausführungsformen der Erfindung; und
- Figur 7: ein Ausschnitt aus Figur 6 im Bereich des Überstands des Docht-Kragens über die Durchgangsöffnung des Trägers.

Der Inhalator 10, hier ein elektronisches Zigarettenprodukt, umfasst ein Gehäuse 11, in dem ein Luftkanal 30 zwischen mindestens einer Lufteinlassöffnung 31 und einer Luftauslassöffnung 24 an einem Mundende 32 des Zigarettenprodukts 10 vorgesehen ist. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt.

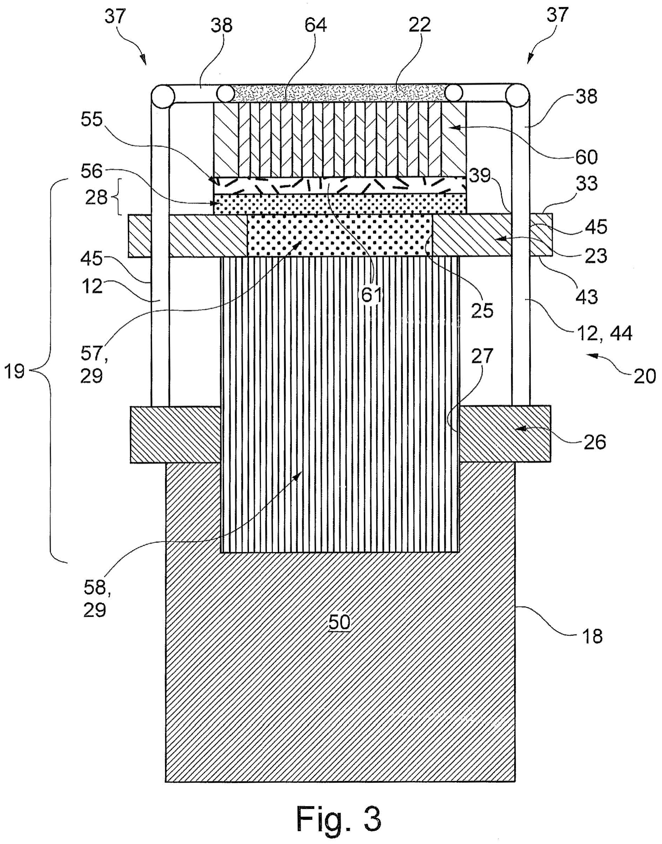

Das Zigarettenprodukt 10 besteht vorteilhaft aus einem Basisteil 16 und einer Verbrauchseinheit 17, die die Verdampfereinheit 20 und den Flüssigkeitsspeicher 18 umfasst und insbesondere in Form einer auswechselbaren Kartusche ausgebildet ist. Die durch die Einlassöffnung 31 angesaugte Luft wird in dem Luftkanal 30 zu oder entlang mindestens einer Verdampfereinheit 20 geleitet. Die Verdampfereinheit 20 ist mit mindestens einem Flüssigkeitsspeicher 18 verbunden oder verbindbar, in dem mindestens eine Flüssigkeit 50 gespeichert ist. Die Verdampfereinheit 20 verdampft Flüssigkeit 50, die ihr aus dem Flüssigkeitsspeicher 18 zugeführt wird, und gibt die verdampfte Flüssigkeit als Aerosol/Dampf 22 (siehe Figur 3) an einer Auslassseite 64 in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

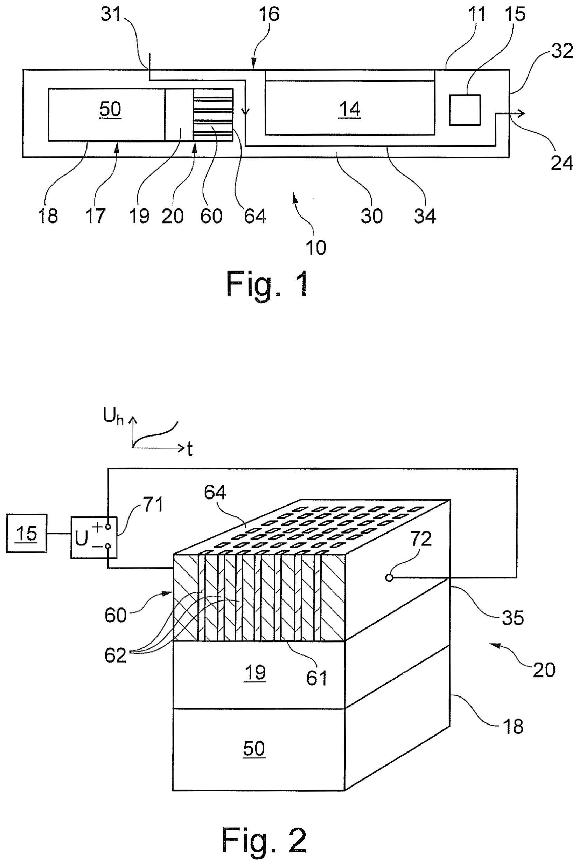

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-Ionen-Akku, sein. Die elektronische Steuerungsvorrichtung 15 umfasst mindestens eine digitale Datenverarbeitungseinrichtung, insbesondere Mikroprozessor und/oder Mikrocontroller, in dem Basisteil 16 (wie in Figur 1 gezeigt) und/oder in der Verbrauchseinheit 17.

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Zigarettenprodukts 10 zieht, um zu inhalieren. In diesem Fall steuert die Steuerungsvorrichtung 15 die Verdampfereinheit 20 an, um Flüssigkeit 50 aus dem Flüssigkeitsspeicher 18 als Aerosol/Dampf in den Luftstrom 34 zuzugeben.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff insbesondere Nikotin.

Die Verbrauchseinheit bzw. Kartusche 17 umfasst vorteilhaft einen nichtflüchtigen Datenspeicher zum Speichern von die Verbrauchseinheit bzw. Kartusche 17 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft über Kontakte und/oder Leitungen mit der Steuereinrichtung 15 verbunden oder verbindbar.

Eine Ausführungsform einer erfindungsgemäßen Verdampfereinheit 20 ist in Fig. 2 gezeigt. Die Verdampfereinheit 20 umfasst einen blockförmigen, vorzugsweise monolithischen, Heizkörper 60, nämlich aus einem elektrisch leitenden Material, nämlich Silizium.

Der Heizkörper 60 ist mit einer Mehrzahl von Mikrokanälen 62 versehen, die eine Einlassseite 61 des Heizkörpers 60 mit einer Auslassseite 64 flüssigkeitsleitend verbinden. Die Einlassseite 61 ist über eine Dochtstruktur 19 flüssigkeitsleitend mit dem Flüssigkeitsspeicher 18 verbunden. Die Dochtstruktur 19 dient zur passiven Förderung von Flüssigkeit aus einem Flüssigkeitsspeicher 50 zu dem Heizkörper 60 mittels Kapillarkräften. Die Dochtstruktur 19 im Kontaktbereich 35, 61 zu dem Heizkörper 60 dient dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Heizkörper 60 in die Dochtstruktur 19 und/oder in den Flüssigkeitsspeicher 18 zu verhindern.

Der mittlere Durchmesser der Mikrokanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Mikrokanal 62 eindringende Flüssigkeit durch den Mikrokanal 62 nach oben steigt, bis der Mikrokanal 62 mit Flüssigkeit gefüllt ist. Das Volumenverhältnis von Mikrokanälen 62 zu Heizkörper 60, das als Porosität des Heizkörpers 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Mikrokanälen 62 versehenen Flächen des Heizkörpers 60 liegen beispielsweise im Bereich zwischen 0,5 mm und 3 mm. Die Abmessungen der mit Mikrokanälen 62 versehenen Flächen des Heizkörpers 60 können beispielsweise betragen:
0,95 mm x 1,75 mm; 1,9 mm x 1,75 mm oder 1,9 mm x 0,75 mm. Die Kantenlängen des Heizkörpers 60 können beispielsweise im Bereich zwischen 0,5 mm und 5 mm liegen, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm x 3 mm oder 2 mm x 3 mm betragen.

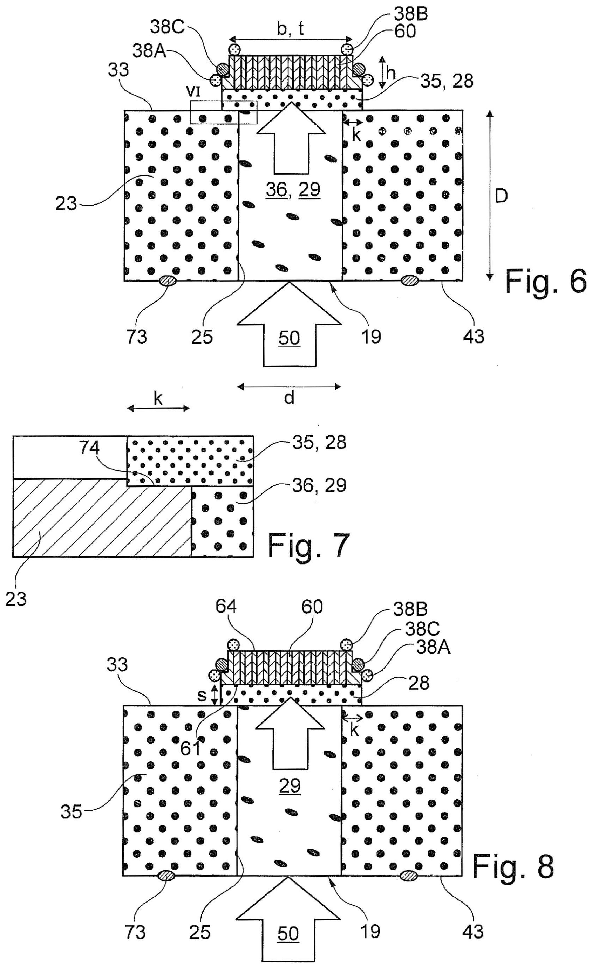

Die Breite b des Heizkörpers 60 (siehe Figur 6) liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörpers 60 (siehe Figur 6) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 62 liegt vorzugsweise im Bereich zwischen vier und 1000. Auf diese Weise lässt sich der Wärmeeintrag von dem Träger in die Mikrokanäle 62 optimieren und eine gesicherte hohe Verdampfungsleistung sowie eine ausreichend große Dampfaustrittsfläche realisieren.

Die Mikrokanäle 62 sind in Form eines quadratischen, rechteckigen, vieleckigen, runden, ovalen oder anders geformten Arrays angeordnet, wie in Figur 3 ersichtlich ist. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 62 mit gesichert hoher Verdampfungsleistung realisieren.

Der Querschnitt der Mikrokanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von dem Heizkörper 60 in die Mikrokanäle 62 realisieren.

Der Abstand zweier Mikrokanäle 62 beträgt vorzugsweise mindestens das 1,3-fache des lichten Durchmessers eines Mikrokanals 62, wobei der Abstand auf die Mittelachsen der beiden Mikrokanäle 62 bezogen ist. Der Abstand kann bevorzugt das 1,5- bis 5-fache, weiter bevorzugt das 2- bis 4-fache des lichten Durchmessers eines Mikrokanals 62 betragen. Auf diese Weise lässt sich ein optimaler Wärmeeintrag von dem Träger in die Mikrokanäle und eine ausreichend stabile Anordnung und Wandstärke der Mikrokanäle realisieren.

Die Verdampfereinheit 20 weist eine vorzugsweise von der Steuerungsvorrichtung 15 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohm'schen Widerstands des elektrisch leitenden Heizkörpers 60 führt der Stromfluss zu einer Erhitzung des Heizkörpers 60 und daher zu einer Verdampfung von in den Mikrokanälen 62 enthaltener Flüssigkeit. Der Heizkörper 60 wirkt somit als Verdampfer. Auf diese Weise erzeugter Dampf/Aerosol entweicht zur Auslassseite 64 aus den Mikrokanälen 62 und wird der Luftströmung 34 beigemischt, siehe Figur 1. Genauer steuert bei Feststellung eines durch Ziehen des Konsumenten verursachten Luftstroms 34 durch den Luftkanal 30 die Steuerungsvorrichtung 15 die Heizspannungsquelle 71 an, wobei durch spontane Erhitzung die in den Mikrokanälen 62 befindliche Flüssigkeit in Form von Dampf/Aerosol aus den Mikrokanälen 62 getrieben wird.

Vorzugsweise ist in dem Datenspeicher des Inhalators 10 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Mikrokanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100°C und 400°C, weiter bevorzugt zwischen 150°C und 350°C, noch weiter bevorzugt zwischen 190°C und 290°C.

Der Heizkörper 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise kleiner oder gleich 750 µm, noch weiter vorzugsweise kleiner oder gleich 500 µm aufweist. Oberflächen des Heizkörpers 60 können vorteilhaft hydrophil sein. Die Auslassseite 64 des Heizkörpers 60 kann vorteilhaft mikrostrukturiert sein bzw. Mikroausnehmungen (micro grooves) aufweisen.

Die Verdampfereinheit 20 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfereinheit 20 hinsichtlich der Flüssigkeits-/Dampfmenge pro Zug einstellbar sein.

An der Einlassseite 61 des Heizkörpers 60 ist eine poröse und/oder kapillare, flüssigkeitsleitende Dochtstruktur 19 angeordnet. Die Dochtstruktur 19 kontaktiert die Einlassseite 61 des Heizkörpers 60 flächig und deckt sämtliche Mikrokanäle 62 einlassseitig ab, wie in Figuren 2, 3, 6 und 8 ersichtlich ist. An der dem Heizkörper 60 gegenüberliegenden Seite ist die Dochtstruktur flüssigkeitsleitend mit dem Flüssigkeitsspeicher verbunden. Die in den Figuren 1 bis 3 gezeigte direkte Anbindung des Flüssigkeitsspeichers 18 an die Dochtstruktur 19 ist nur beispielhaft zu verstehen. Insbesondere können eine Flüssigkeitsschnittstelle und/oder eine mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Der Flüssigkeitsspeicher 18 kann in seinen Abmessungen größer als die Dochtstruktur 19 sein, siehe beispielsweise Figur 3. Die Dochtstruktur 19 kann beispielsweise in eine Öffnung eines Gehäuses des Flüssigkeitsspeichers 18 eingesetzt sein. Es kann auch eine Mehrzahl von Verdampfereinheiten 20 einem Flüssigkeitsspeicher 18 zugeordnet sein.

Die Dochtstruktur 19 besteht aus porösem und/oder kapillarem Material, das aufgrund von Kapillarkräften in der Lage ist, von dem Heizkörper 60 verdampfte Flüssigkeit in ausreichender Menge von dem Flüssigkeitsspeicher 18 zu dem Heizkörper 60 passiv nachzufördern, um ein Leerlaufen der Mikrokanäle 62 und sich daraus ergebende Probleme zu verhindern.

Die Dochtstruktur 19 besteht vorteilhaft aus einem nichtleitenden Material, um eine unerwünschte Erwärmung von Flüssigkeit in der Dochtstruktur 19 durch Stromfluss zu vermeiden. Falls die Dochtstruktur 19 aus einem leitenden Material besteht, was nicht ausgeschlossen ist, ist zwischen der Dochtstruktur 19 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Mikrokanälen 62 korrespondierenden Durchgangsöffnungen vorgesehen.

Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm³ und 10 mm³, weiter vorzugsweise im Bereich zwischen 2 mm³ und 8 mm³, noch weiter vorzugsweise im Bereich zwischen 3 mm³ und 7 mm³ und beträgt beispielsweise 5 mm³.

Vorteilhafte Ausführungsformen einer Verdampfereinheit 20 sind in den Figuren 3 bis 8 gezeigt. Die Dochtstruktur 19 kann generell einteilig, siehe Figur 8, oder mehrteilig sein, siehe Figuren 3 und 6.

In der Ausführungsform nach Figur 6 ist die Dochtstruktur 19 beispielsweise zweischichtig mit einer Dochtschicht 35, die an der Einlassseite 61 des Heizkörpers 60 flächig kontaktierend anliegt, und einer daran flächig anliegenden weiteren Dochtschicht 36. Die Dochtschicht 35 kann bevorzugt eine Faserpapier- oder Keramikpapierschicht, mit oder ohne Glassfilter-Filter, sein. Die Dochtschicht 36 kann bevorzugt eine poröse Keramik sein.

Im Ausführungsbeispiel nach Figur 3 umfasst die Dochtstruktur 19 mehr als zwei, vier beispielsweise Schichten. Unmittelbar angrenzend an den Heizkörper 60, und diesen flächig kontaktierend, ist eine Filterschicht 55 angeordnet, die insbesondere aus einer, zwei oder mehr Mikroglasfaser-Lagen bestehen kann. Daran flächig angrenzend kann eine Faserpapier-Schicht 56 angeordnet sein. Daran flächig angrenzend sind vorteilhaft Dochtschichten 57, 58 vorgesehen, beispielsweise eine Keramikdocht-Schicht 57 und eine Öllampendocht-Schicht 58, d.h. ein Glasfaser-Dochtmaterial, das herkömmlich für die Dochte von Öllampen verwendet wird.

Die Kapillarkräfte für die Kapillarförderung der Flüssigkeit von dem Flüssigkeitsspeicher 18 zu dem Heizkörper 60 können überwiegend oder vollständig von den Dochtschichten 57, 58 bereitgestellt werden. Es ist im Allgemeinen nicht erforderlich, wenn sämtliche Schichten der Dochtstruktur 19 Kapillarkräfte für die Kapillarförderung der Flüssigkeit bereitstellen. Es kann auch ausreichen, dass nur eine Schicht der Dochtstruktur 19 Kapillarkräfte für die Kapillarförderung der Flüssigkeit bereitstellt.

Die Verdampfereinheit 20 weist einen insbesondere plattenförmigen Träger 23 zum Halten des Heizkörpers 19 und/oder der Dochtstruktur 19 auf, wie in den Figuren 3 bis 8 gezeigt ist. Der Träger 23 kann aus einem geeigneten Material, beispielsweise Keramik, Glas und/oder Kunststoff einschließlich faserverstärktem Kunststoff, beispielsweise Leiterplattenmaterial bestehen und weist eine Durchgangsöffnung 25 auf, durch die sich die Dochtstruktur 19 erstreckt und in der die Dochtstruktur 19 gehalten ist.

Die Dicke D des Trägers 23 (siehe Figur 6) liegt vorzugsweise im Bereich zwischen 0,5 mm bis 4 mm, weiter vorzugsweise im Bereich zwischen 1 mm bis 3 mm, noch weiter vorzugsweise im Bereich zwischen 1 mm und 2 mm und kann beispielsweise 1,6 mm oder 2 mm betragen. Die Dicke einer in der Durchgangsöffnung 25 des Trägers 23 angeordneten Dochtschicht 57 kann an die Dicke des Trägers 23 angepasst sein bzw. dieser entsprechen und daher beispielsweise ebenfalls 1.6 mm oder 2 mm betragen.

Die Durchgangsöffnung 25 ist vorteilhaft kreisrund, was einfach zu fertigen ist. Der Durchmesser d, oder ggf. der mittlere Durchmesser, der Durchgangsöffnung 25 (siehe Figur 6) liegt vorzugsweise im Bereich zwischen 0,5 mm und 4 mm, vorzugsweise im Bereich zwischen 1 mm bis 3 mm, weiter vorzugsweise im Bereich zwischen 1,5 mm und 2,5 mm und beträgt beispielsweise 2 mm.

Der Durchmesser d der Durchgangsöffnung 25 ist kleiner oder gleich, vorteilhaft kleiner als die Breite b des Heizkörpers 60, siehe Figur 6. Das Volumen der Durchgangsöffnung 25, bzw. das Dochtvolumen in der Durchgangsöffnung 25, liegt vorteilhaft im Bereich zwischen 1 mm³ und 8 mm³, vorzugsweise im Bereich zwischen 2 mm³ und 6,5 mm³, weiter vorzugsweise im Bereich zwischen 2,5 mm³ und 5 mm³.

Die Dochtstruktur 19 weist einen Kragenförmigen Abschnitt oder Kragen 28 und einen Schaftabschnitt oder Schaft 29 auf, oder besteht aus diesen Komponenten 28, 29.

Der Kragen 28 ist zwischen dem Heizkörper 60 und dem Träger 23 angeordnet, liegt an dem Heizkörper 60 an der Einlassseite 61 flächig kontaktierend an und bedeckt dabei sämtliche Mikrokanäle 62. Die Dicke s des Kragens (siehe Figur 8) liegt vorteilhaft im Bereich zwischen 0,05 mm und 1 mm und beträgt vorzugsweise höchstens 0,8 mm, weiter vorzugsweise höchsten 0,6 mm noch weiter vorzugsweise höchsten 0,4 mm und beispielsweise 0,2 mm.

Der Schaftabschnitt 29 liegt an dem Kragen 28 an dessen dem Heizkörper 60 abgewandter Seite flächig an. Die reelle oder gedachte Trennfläche zwischen Kragen 28 und Schaftabschnitt 29 kann mit der dem Heizkörper 60 zugewandten Oberfläche des Trägers 23 in einer Ebene liegen. Der Schaftabschnitt 29 kann insbesondere den übrigen Teil des Dochtabschnitts 19, abgesehen von dem Kragen 28, bezeichnen. Der Schaftabschnitt 29 kann im freien, vormontierten Zustand ein Übermaß, d.h. einen größeren Durchmesser haben als die Durchgangsöffnung 25, um zusätzliche Haltekräfte des Schafts 29 in der Durchgangsöffnung 25 zu erzeugen.

Kragen 28 und Schaft 29 können einteilig bzw. einstückig gebildet sein, siehe Figur 8. Kragen 28 und Schaft 29 können auch jeweils eigene Teile sein und von entsprechenden Dochtabschnitten 35, 36 gebildet sein, siehe Figur 6. Figur 3 verdeutlicht, dass Kragen 28 und/oder Schaft 29 jeweils mehrteilig ausgebildet und jeweils von entsprechenden Dochtabschnitten 55, 56 bzw. 57, 58 gebildet sein können.

Der Durchmesser t des Kragens 28 (siehe Figur 6) ist größer als der Durchmesser d der Durchgangsöffnung 25 und somit des Schafts 29 im Bereich der Durchgangsöffnung 25. Vorzugsweise steht der Kragen 28 über seinen gesamten Umfang über die Durchgangsöffnung 25 mit Überstand k über. Der Überstand k des Kragens 28 über die Durchgangsöffnung 25 beträgt vorzugsweise mindestens 0,1 mm, weiter vorzugsweise mindestens 0,2 mm, noch weiter vorzugsweise mindestens 0,3 mm und besonders bevorzugt mindestens 0,4 mm.

Aufgrund des allseitigen Überstands des Kragens 28 über die Durchgangsöffnung 25 wird bei einer Klemmung des Heizkörpers 60 auf den Träger 23 der Kragen 28, und somit die gesamte Dochtstruktur 19, sicher in der Verdampfereinheit 20 gehalten.

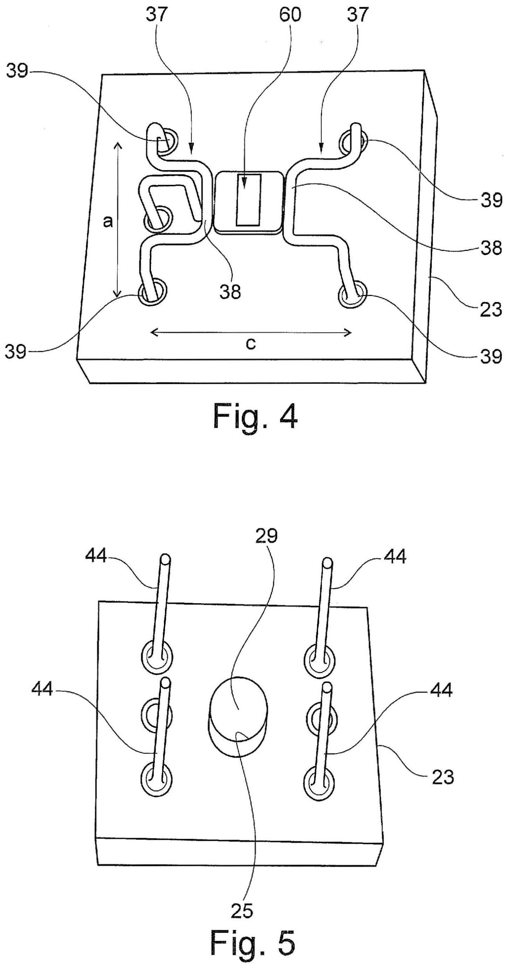

Die Klemmung des Heizkörpers 60 auf dem Träger 23 wird mittels mindestens zwei Klemmelementen 37 bewirkt, siehe insbesondere Figur 4, die an gegenüberliegenden Seiten des Heizkörpers 60 an diesem angreifen. Jedes Klemmelement 37 weist vorteilhaft einen Klemmbügel 38 auf, der an zwei voneinander beabstandeten Befestigungspunkten 39 federnd an dem Träger 23 befestigt ist und eine Vorspannung erzeugt, mittels der der Heizkörper 60 und der Kragen 28 auf dem Träger 23 festgeklemmt wird.

Der Abstand a der beiden Befestigungspunkte 39 eines Klemmbügels 38 voneinander liegt vorzugsweise im Bereich zwischen 4 mm und 10 mm, weiter vorzugsweise im Bereich zwischen 5 mm bis 8 mm und beträgt beispielsweise 6 mm. Der Abstand c zwischen den Befestigungspunkten 39 zweier Klemmbügel 39 voneinander liegt vorzugsweise im Bereich zwischen 5 mm und 12 mm, weiter vorzugsweise im Bereich zwischen 6 mm bis 10 mm und beträgt beispielsweise 8 mm. Die Abmessungen des beispielsweise rechteckigen Trägers 23 liegen vorzugsweise im Bereich zwischen 6 mm und 20 mm, weiter vorzugsweise im Bereich zwischen 8 mm bis 17 mm und noch weiter vorzugsweise im Bereich zwischen 10 mm und 14 mm.

Besonders vorteilhaft dienen die Klemmelemente 37 gleichzeitig als Elektroden zur Kontaktierung des Heizkörpers 60 und dessen Versorgung mit Heizstrom. Zu diesem Zweck bestehen die Klemmelemente 37 bzw. die Klemmbügel 38 vorteilhaft aus einem elektrisch leitenden Material, beispielsweise kann es sich um Metalldraht, beispielsweise Messingdraht handeln. Aufgrund der Linienkontaktierung zwischen dem Klemmbügel 38 und dem Heizkörper 60 ergibt sich eine ausgezeichnete elektrische Verbindung zwischen dem Klemmelement 37 und dem Heizkörper 60, bei gleichzeitig idealer thermischer Entkopplung zwischen dem Klemmelement 37 und dem Heizkörper 60 wegen fehlendem Flächenkontakt. Wärmedissipation von dem Heizkörper 60 in das Klemmelement 37 ist daher gering, die Elektroden 38 bleiben signifikant kühler als der Heizkörper 60.

Der Klemmbügel 38 kann den Heizkörper 60 seitlich parallel zur Auslassseite 64 (Position 38A in Figur 6) und/oder senkrecht auf die Auslassseite 64 (Position 38B in Figur 6) und/oder in einer Nut oder Stufe mit einem Zwischenwinkel, beispielsweise zwischen 30° und 60°, sowohl seitlich als auch senkrecht auf die Auslassseite 64 klemmen (Position 38C in Figur 6). Die letztgenannte Möglichkeit involviert zwei Kontaktlinien zwischen dem Klemmbügel 38C und dem Heizkörper 60, was die elektrische Kontaktierung weiter verbessert. Ein Klemmelement 37 kann auch mehr als einen Klemmbügel 38 aufweisen, insbesondere beliebige zwei oder alle drei der Klemmbügel 38A, 38B, 38C.

Die Klemmelemente 37 sind mittels elektrischer Leitungen 12 vorteilhaft mit einer in der Verbrauchseinheit 17 vorgesehenen Leiterplatte 26 (PCB) verbunden, um die elektrische Verbindung zu der elektronischen Steuerungsvorrichtung 15 und zu der Energiequelle 46 für die Stromversorgung des Heizkörpers 60 herzustellen. Auf der Leiterplatte 26 sind vorteilhaft elektronische Komponenten der Verbrauchseinheit 17 angeordnet.

Die Leiterplatte 26 ist in dem Ausführungsbeispiel gemäß Figur 3 ein separates Teil und von dem Träger 23 beabstandet auf dessen dem Heizkörper 60 abgewandter Seite 43 angeordnet. Die Leiterplatte 26 weist eine Durchgangsöffnung 27 auf, durch die sich der Schaft 29 der Dochtstruktur 19 erstreckt und in der die Dochtstruktur 19 gehalten sein kann. Die elektrischen Leitungen 12 umfassen hier beispielsweise vier Metallstifte 44, die auf der Seite 33 des Trägers 23 in den Befestigungspunkten 39 mit den Klemmelementen 37 verbunden und jeweils durch eine Durchgangsbohrung 45 durch den Träger 23 hindurchgeführt sind und dann auf der abgewandten Seite 43 den Abstand zwischen dem Träger 23 und der Leiterplatte 26 überbrücken.

In einer anderen Ausführungsform kann der Träger 23 die Leiterplatte 26 ausbilden. Die elektrischen Leitungen 12 können dann entfallen. Es ist auch möglich, dass die Verdampfereinheit 20 selbst keine Leiterplatte umfasst, sondern die Klemmbügel 38 beispielsweise über flexible isolierte Leitungen 12, oder auf andere geeignete Weise, mit einer etwa in dem Basisteil 16 angeordneten Leiterplatte verbunden sind.

An der Oberseite 33 des Trägers 23 kann eine an den Kragen 28 angepasste Ausnehmung 74 vorgesehen sein, in die der Kragen 28 bei der Montage passgenau einlegbar ist, um eine optimale Montageposition des Kragens 28 zu definieren.

An der Unterseite 43 des Trägers 23 kann ein Dichtelement 73, beispielsweise ein Dichtring, zur Abdichtung des Trägers 23 gegen ein Gehäuse des Flüssigkeitsspeichers 18, oder einer anderen unter dem Träger 23 angeordneten Komponente, angeordnet sein, siehe Figuren 6 und 8.

Die von der Heizspannungsquelle 71 erzeugte Ansteuerfrequenz des Heizkörpers 60 liegt vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Im Folgenden wird der Ablauf des Verdampfungsvorgangs erläutert.

In einem Ausgangszustand ist die Spannungsquelle 71 für den Heizvorgang ausgeschaltet.

Zum Verdampfen von Flüssigkeit 50 wird die Spannungsquelle 71 für den Heizkörper 60 aktiviert. Die Spannung Uh wird dabei so eingestellt, dass die Verdampfungstemperatur in dem Heizkörper 60 und somit in den Mikrokanälen 62 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der Mikrokanäle 62 entspricht oder damit in Zusammenhang steht, wird die Heizspannungsquelle 71 deaktiviert. Da die Liquideigenschaften und -menge vorteilhaft exakt bekannt sind, kann dieser Zeitpunkt sehr genau gesteuert werden. Die Energieaufnahme der Verdampfereinheit 20 lässt sich daher gegenüber bekannten Vorrichtungen reduzieren, da die benötigte Verdampfungsenergie dosierter und damit exakter eingebracht werden kann. Aufgrund der Ausbildung des Heizkörpers 60 kann dieser auch als Volumenverdampfer bezeichnet werden, im Gegensatz zu herkömmlichen Flächenverdampfern.

Nach Abschluss des Heizvorgangs sind die Mikrokanäle 62 überwiegend oder vollständig entleert. Die Heizspannung 71 wird dann so lange ausgeschaltet gehalten, bis mittels Nachförderung von Flüssigkeit durch die Dochtstruktur 19 die Mikrokanäle 62 wieder aufgefüllt sind. Sobald dies der Fall ist, kann der nächste Heizzyklus durch Einschalten der Heizspannung 71 begonnen werden.

Die Verdampfereinheit 20 ist vorzugsweise auf der Grundlage von MEMS-Technologie, insbesondere aus Silizium, gefertigt und daher vorteilhaft ein Mikro-Elektro-Mechanisches System.

## Patentansprüche

1. Verdampfereinheit für einen Inhalator, insbesondere für ein elektronisches Zigarettenprodukt, mit einem elektrisch betreibbaren, insbesondere planaren Heizkörper (60), der eine Einlassseite (61) und eine Auslassseite (64) aufweist, einem Träger (23) zum Tragen des Heizkörpers, und einer Mehrzahl von Mikrokanälen (62), die sich jeweils von der Einlassseite (61) zu der Auslassseite (64) durch den Heizkörper (60) erstrecken, wobei der Heizkörper (60) durch Anlegen einer Heizspannung zum Verdampfen von durch die Mikrokanäle (62) geförderter Flüssigkeit eingerichtet ist, wobei an der Einlassseite (61) des Heizkörpers (60) eine poröse und/oder kapillare Dochtstruktur (19) angeordnet ist, die flüssigkeitsleitend mit einem Flüssigkeitsspeicher (18) verbunden oder verbindbar ist, wobei die Dochtstruktur (19) einen sich durch eine Durchgangsöffnung (25) des Trägers (23) erstreckenden Schaft und einen zwischen dem Träger (23) und dem Heizkörper (60) angeordneten umlaufenden Kragen (28) aufweist, wobei der Durchmesser des Kragens (28) größer ist als der Durchmesser der Durchgangsöffnung (25) des Trägers (23), **dadurch gekennzeichnet, dass** der Kragen (28) über seinen gesamten Umfang über die Durchgangsöffnung (25) des Trägers (23) übersteht, wobei der Heizkörper (60) blockförmig und aus einem elektrisch leitenden Material, nämlich Silizium, ist.

2. Verdampfereinheit nach Anspruch 1, **dadurch gekennzeichnet, dass** die Verdampfereinheit (20) mindestens ein eine Vorspannung erzeugendes Klemmelement (37) aufweist, das zur Klemmung des Heizkörpers (60) und des Kragens (28) auf den Träger (23) angeordnet und eingerichtet ist.

3. Verdampfereinheit nach Anspruch 2, **dadurch gekennzeichnet, dass** mindestens zwei Klemmelemente (37) auf entgegengesetzten Seiten des Heizkörpers (60) vorgesehen sind.

4. Verdampfereinheit nach Anspruch 2 oder 3, **dadurch gekennzeichnet, dass** das mindestens eine Klemmelement (37) einen den Heizkörper (60) linienförmig kontaktierenden Klemmbügel (38) aufweist.

5. Verdampfereinheit nach einem der Ansprüche 2 bis 4, **dadurch gekennzeichnet, dass** das mindestens eine Klemmelement (37) den Heizkörper (60) seitlich parallel zur Auslassseite und/oder senkrecht auf die Auslassseite (64) und/oder in einer Nut oder Stufe des Trägers (23) klemmt.

6. Verdampfereinheit nach einem der Ansprüche 2 bis 5, **dadurch gekennzeichnet, dass** das mindestens eine Klemmelement (37) als Elektrode zur elektrischen Kontaktierung und Versorgung des Heizkörpers (60) dient.

7. Verdampfereinheit nach Anspruch 6, **dadurch gekennzeichnet, dass** mindestens ein sich durch eine Bohrung (45) des Trägers (23) erstreckender Leiter (12) zur Kontaktierung des Klemmelements (37) vorgesehen ist.

8. Verdampfereinheit nach Anspruch 7, **dadurch gekennzeichnet, dass** der mindestens eine Leiter (12) eine Leiterplatte (26) kontaktiert, die auf der von dem Heizkörper (60) abgewandten Seite des Trägers (23) beabstandet angeordnet ist.

9. Verdampfereinheit nach einem der vorangehenden Ansprüche, **dadurch gekennzeichnet, dass** der Träger (23) als Leiterplatte ausgebildet ist.

10. Verdampfereinheit nach einem der vorangehenden Ansprüche, **dadurch gekennzeichnet, dass** der allseitige Überstand k des Kragens (28) über die Durchgangsöffnung (25) des Trägers (23) mindestens 0,1 mm beträgt.

11. Verdampfereinheit nach einem der vorangehenden Ansprüche, **dadurch gekennzeichnet, dass** die Förderrate der Dochtstruktur (19) mindestens so groß ist wie die maximale Verdampfungsrate des Heizkörpers (60).

12. Verdampfereinheit nach einem der vorangehenden Ansprüche, **dadurch gekennzeichnet, dass** die Dochtstruktur (19) aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien besteht.

13. Verdampfereinheit nach einem der vorangehenden Ansprüche, **dadurch gekennzeichnet, dass** die Dochtstruktur (19) eine Filterschicht (55) insbesondere aus Mikroglasfaser aufweist.

14. Verdampfereinheit nach einem der vorangehenden Ansprüche, **dadurch gekennzeichnet, dass** die Dochtstruktur (19) eine Faserpapier- und/oder Keramikpapierschicht (35; 56) aufweist.

15. Verdampfereinheit nach einem der vorangehenden Ansprüche, **dadurch gekennzeichnet, dass** die Dochtstruktur (19) eine poröse Keramikschicht (36; 57) aufweist.

16. Verdampfereinheit nach einem der vorangehenden Ansprüche, **dadurch gekennzeichnet, dass** die Dochtstruktur (19) eine Öllampendocht-Schicht (58) aufweist.

## Claims

1. Evaporator unit for an inhaler, in particular for an electronic cigarette product, comprising an electrically operable heating body (60), in particular a flat heating body, which has an inlet side (61) and an outlet side (64), a support (23) for supporting the heating body, and a plurality of microchannels (62), each of which extends from the inlet side (61) to the outlet side (64) through the heating body (60), wherein the heating body (60) is designed to evaporate liquid being transferred through the microchannels (62) by applying a heating voltage, wherein a porous and/or capillary wick structure (19) is arranged on the inlet side (61) of the heating body (60), said wick structure being fluidically connected or connectable to a liquid store (18), wherein the wick structure (19) has a shaft which extends through a passage opening (25) of the support (23), and a circumferential collar (28), which is arranged between the support (23) and the heating body (60), wherein the diameter of the collar (28) is greater than the diameter of the passage opening (25) of the support (23), **characterised in that** the collar (28) protrudes over its entire circumference over the passage opening (25) of the support (23), wherein the heating body (60) is block-shaped and composed of an electrically conductive material, specifically silicon.

2. Evaporator unit according to claim 1, **characterised in that** the evaporator unit (20) has at least one clamping element (37) which generates a pretension and which is arranged and set up for clamping the heating body (60) and the collar (28) onto the support (23).

3. Evaporator unit according to claim 2, **characterised in that** at least two clamping elements (37) are provided on opposite sides of the heating body (60).

4. Evaporator unit according to either claim 2 or claim 3, **characterised in that** the at least one clamping element (37) has a clamping bracket (38) which makes linear contact with the heating body (60).

5. Evaporator unit according to any of claims 2 to 4, **characterised in that** the at least one clamping element (37) clamps the heating body (60) laterally parallel to the outlet side and/or perpendicularly to the outlet side (64) and/or in a groove or step of the support (23).

6. Evaporator unit according to any of claims 2 to 5, **characterised in that** the at least one clamping element (37) serves as an electrode for electrically contacting and supplying the heating body (60).

7. Evaporator unit according to claim 6, **characterised in that** at least one conductor (12) extending through a bore (45) in the support (23) is provided for contacting the clamping element (37).

8. Evaporator unit according to claim 7, **characterised in that** the at least one conductor (12) contacts a circuit board (26) which is arranged at a distance on the side of the support (23) facing away from the heating body (60).

9. Evaporator unit according to any of the preceding claims, **characterised in that** the support (23) is designed as a circuit board.

10. Evaporator unit according to any of the preceding claims, **characterised in that** the all-round protrusion k of the collar (28) over the passage opening (25) of the support (23) is at least 0.1 mm.

11. Evaporator unit according to any of the preceding claims, **characterised in that** the transfer rate of the wick structure (19) is at least as large as the maximum evaporation rate of the heating body (60).

12. Evaporator unit according to any of the preceding claims, **characterised in that** the wick structure (19) consists of one or a plurality of the following materials: cotton, cellulose, acetate, glass fibre fabric, glass fibre ceramic, sintered ceramic, ceramic paper, aluminosilicate paper, metal foam, metal sponge, another heat-resistant, porous and/or capillary material having a suitable transfer rate, or a combination of two or a plurality of the materials mentioned above.

13. Evaporator unit according to any of the preceding claims, **characterised in that** the wick structure (19) has a filter layer (55), in particular made of micro-glass fibre.

14. Evaporator unit according to any of the preceding claims, **characterised in that** the wick structure (19) has a fibre paper and/or a ceramic paper layer (35; 56).

15. Evaporator unit according to any of the preceding claims, **characterised in that** the wick structure (19) has a porous ceramic layer (36; 57).

16. Evaporator unit according to any of the preceding claims, **characterised in that** the wick structure (19) has an oil lamp wick layer (58).

## Revendications

1. Unité de vaporisation pour un inhalateur, en particulier pour un produit cigarette électronique, comportant un corps chauffant (60) pouvant fonctionner électriquement, en particulier plan, qui présente un côté entrée (61) et un côté sortie (64), un support (23) pour supporter le corps chauffant et une pluralité de microcanaux (62) s'étendant chacun à travers le corps chauffant (60) depuis le côté entrée (61) jusqu'au côté sortie (64), le corps chauffant (60) étant conçu pour vaporiser le liquide transporté à travers les microcanaux (62) par application d'une tension de chauffage, une structure de mèche (19) poreuse et/ou capillaire étant disposée du côté entrée (61) du corps chauffant (60), laquelle est reliée ou peut être reliée de manière conductrice de liquide à un réservoir de liquide (18), la structure de mèche (19) présentant une tige s'étendant à travers une ouverture traversante (25) du support (23) et une collerette (28) circonférentielle disposée entre le support (23) et le corps chauffant (60), le diamètre de la collerette (28) étant supérieur au diamètre de l'ouverture traversante (25) du support (23), **caractérisée en ce que** la collerette (28) dépasse sur toute sa circonférence au-delà de l'ouverture traversante (25) du support (23), le corps chauffant (60) étant en forme de bloc et composé d'un matériau électriquement conducteur, à savoir du silicium.

2. Unité de vaporisation selon la revendication 1, **caractérisée en ce que** l'unité de vaporisation (20) présente au moins un élément de serrage (37) générant une précontrainte, qui est disposé et conçu pour serrer le corps chauffant (60) et la collerette (28) sur le support (23).

3. Unité de vaporisation selon la revendication 2, **caractérisée en ce qu'**au moins deux éléments de serrage (37) sont prévus sur des côtés opposés du corps chauffant (60).

4. Unité de vaporisation selon la revendication 2 ou 3, **caractérisée en ce que** ledit au moins un élément de serrage (37) présente un étrier de serrage (38) en contact linéaire avec le corps chauffant (60).

5. Unité de vaporisation selon l'une des revendications 2 à 4, **caractérisée en ce que** ledit au moins un élément de serrage (37) serre le corps chauffant (60) latéralement parallèlement au côté sortie et/ou perpendiculairement au côté sortie (64) et/ou dans une rainure ou un gradin du support (23).

6. Unité de vaporisation selon l'une des revendications 2 à 5, **caractérisée en ce que** ledit au moins un élément de serrage (37) sert d'électrode pour la mise en contact et l'alimentation électrique du corps chauffant (60).

7. Unité de vaporisation selon la revendication 6, **caractérisée en ce qu'**au moins un conducteur (12) s'étendant à travers un alésage (45) du support (23) est prévu pour la mise en contact avec l'élément de serrage (37).

8. Unité de vaporisation selon la revendication 7, **caractérisée en ce que** ledit au moins un conducteur (12) est en contact avec une carte de circuit imprimé (26) qui est disposée à distance du côté du support (23) opposé au corps chauffant (60).

9. Unité de vaporisation selon l'une des revendications précédentes, **caractérisée en ce que** le support (23) est réalisé sous la forme d'une carte de circuit imprimé.

10. Unité de vaporisation selon l'une quelconque des revendications précédentes, **caractérisée en ce que** le dépassement k de tous les côtés de la collerette (28) au-delà de l'ouverture traversante (25) du support (23) est d'au moins 0,1 mm.

11. Unité de vaporisation selon l'une des revendications précédentes, **caractérisée en ce que** la vitesse de transport de la structure de mèche (19) est au moins aussi grande que la vitesse de vaporisation maximale du corps chauffant (60).

12. Unité de vaporisation selon l'une des revendications précédentes, **caractérisée en ce que** la structure de mèche (19) est constituée d'un ou plusieurs des matériaux suivants : coton, cellulose, acétate, tissu en fibre de verre, céramique en fibre de verre, céramique frittée, papier céramique, papier aluminosilicate, mousse métallique, éponge métallique, un autre matériau résistant à la chaleur, poreux et/ou capillaire ayant une vitesse de transport appropriée, ou un composite de deux ou plusieurs des matériaux précités.

13. Unité de vaporisation selon l'une des revendications précédentes, **caractérisée en ce que** la structure de mèche (19) présente une couche filtrante (55), en particulier en microfibre de verre.

14. Unité de vaporisation selon l'une des revendications précédentes, **caractérisée en ce que** la structure de mèche (19) présente une couche de papier de fibres et/ou de papier céramique (35 ; 56).

15. Unité de vaporisation selon l'une des revendications précédentes, **caractérisée en ce que** la structure de mèche (19) présente une couche de céramique poreuse (36 ; 57).

16. Unité de vaporisation selon l'une des revendications précédentes, **caractérisée en ce que** la structure de mèche (19) présente une couche de mèche de lampe à huile (58).
